# EUROPEAN PATENT SPECIFICATION

(11) **EP 2 785 875 B1**
(45) Date of publication and mention of the grant of the patent: **27.04.2016**
(21) Application number: 12790564.4
(22) Date of filing: 26.11.2012
(51) Int. Cl.: C12Q 1/70

(54) **A SINGLE NUCLEOTIDE POLYMORPHISM ON CHROMOSOME 15 THAT PREDICTS HCV TREATMENT RESPONSES**
EINZELNUKLEOTID-POLYMORPHISMUS AUF CHROMOSOM 15 ZUR VORHERSAGE VON HCV-BEHANDLUNGSREAKTIONEN
POLYMORPHISME DE NUCLÉOTIDE UNIQUE SUR LE CHROMOSOME 15 QUI PRÉDIT LES SENSIBILITÉS VIS-À-VIS D'UN TRAITEMENT CONTRE LE VHC

(30) Priority: 28.11.2011 US 201161564032 P
(43) Date of publication of application: 08.10.2014
(73) Proprietor: F.Hoffmann-La Roche AG, 4070 Basel (CH)
(72) Inventor: BENAYED, Ryma, Clifton, NJ 07013 (US); ESSIOUX, Laurent, 68220 Attenschwiller (FR); NAVARRO, Mercidita T., Santa Clara, CA 95051 (US); PALERMO, Giuseppe, 4058 Basel (CH); RILEY-GILLIS, Bridget, West Orange, NJ 07052 (US); ZHU, Yonghong, Mountain View, CA 94040 (US)
(74) Representative: Halbig, Dirk
(86) International application number: PCT/EP2012/073554
(87) International publication number: WO 2013/079424

(56) References cited:
- WO-A1-2011/058084
- WO-A1-2011/128278
- US-A1- 2010 158 866

## Description

The present invention relates to methods that useful for predicting the response of hepatitis C virus (HCV) infected patients to pharmacological treatment.

Hepatitis C virus (HCV) is responsible for a large proportion of chronic liver disease worldwide and accounts for 70% of cases of chronic hepatitis in industrialized countries. The global prevalence of chronic hepatitis C (CHC) is estimated to average 3% (ranging from 0.1%-5%), with an estimated 170 million chronic carriers worldwide (2.7 million in the USA and 5 million in Western Europe [1, 2]. Approximately one-fifth of chronically infected patients with HCV eventually develop cirrhosis, which may lead to liver failure and hepatocellular carcinoma [2]. HCV-related liver failure is one of the primary reasons for liver transplantation today.

The current standard of care (SOC) for treatment-naive patients with chronic hepatitis C infection is combination therapy with polyethylene glycol conjugated interferon alpha (PEG-IFN) plus ribavirin (RBV) [3]. In patients chronically infected with HCV genotype 1, which represents the majority of HCV-infected patients, responses to PEG-IFN remain sub-optimal with sustained virologic response (SVR) rates between 42-52% [4, 5. 6]. There is a substantial need for new therapeutic options for this patient population. Interferon alpha (IFN) was the first drug shown to have bioactivity against HCV. Hoffmann-La Roche Inc. has chemically modified the interferon alfa-2a molecule by covalently attaching a branched methoxy polyethylene glycol moiety [9]. PEG-IFN has a decreased systemic clearance rate and an approximately 10-fold increase in serum half-life compared with interferon alfa-2a, and as a result PEG-IFN circulates in the blood much longer than the parent compound. Subsequent evaluation of PEG-IFN, 180 ?g once weekly (qw), in three large clinical trials in over 1400 patients showed that treatment with PEG-IFN was more efficacious than treatment with IFN thrice weekly [10].

Ribavirin is a guanosine analogue that inhibits the in vitro replication of a wide range of RNA and DNA viruses [11]. The mechanism by which RBV acts as an antiviral is not fully defined, although it may involve alteration of cellular nucleotide pools and inhibition of viral RNA synthesis [12]. RBV monotherapy has little or no effect on the replication of HCV, but it can result in normalization of serum alanine aminotransferase (ALT) activity and improvement in liver histology. However, relapse occurs in nearly all patients treated with RBV alone [13, 14]. Combining RBV with PEG-IFN has been found to be more effective than PEG-IFN monotherapy in the treatment of CHC. In a large clinical trial of 1121 patients of all genotypes, a sustained virologic response (SVR) was achieved in 53% of patients treated with PEG-IFN plus RBV as compared to 29% of patients treated with PEG-IFN alone [10].

The probability of achieving a Sustained Virological Response (SVR) varies with a collection of patient and viral factors. For example, younger patients, Caucasian and Asian patients, and individuals without advanced hepatic fibrosis are more likely to clear HCV infection after treatment [15-18] Similarly, patients infected with HCV genotypes 2 or 3, rather than genotype 1, and those with low baseline HCV RNA levels in serum have the best chance of a cure [4-6, 16,18].

More precise prediction of SVR is currently possible only after the start of treatment. Regardless of HCV genotype, individuals who clear HCV RNA after 4 or 12 weeks of treatment have a much better chance of achieving an SVR than those with persistent viremia [19]. Rapid virological response (RVR, undetectable HCV RNA at week 4) is a strong predictor of SVR; conversely, failure to achieve an early virological response (EVR, greater than a two log decline in HCV RNA at week 12) is a strong predictor of nonresponse, independent of pretreatment characteristics [20].

The ability to prospectively differentiate between potential responders and non-responders to the standard of care could have a great impact on the care of patients with chronic hepatitis C. In addition to host and viral factors, host genetic diversity also influences the response to treatment with the standard of care [21]. Recent evidence from genome-wide association studies suggests that single nucleotide polymorphisms (SNPs) in the promoter region of the IL-28b gene, exert a strong influence on the probability of SVR in patients treated with peginterferon plus ribavirin [22-24].

Recently, it was discovered that in patients infected with Hepatitis C Virus Genotype 1 (HCV-1) or Genotype 4 (HCV-4), a beneficial response to a treatment that includes interferon alpha, ribavirin and a HCV polymerase inhibitor (Triple Therapy) could be predicted if the patient's HCV RNA level becomes undetectable in as short as two weeks post treatment.

The present invention is based on the discovery of an association between a single nucleotide polymorphism on human chromosome fifteen and RVR2 in patients treated with an interferon-based regimen that included a direct acting antiviral agent, such as an HCV protease inhibitor. In one embodiment, the invention provides for a method for predicting response of a human subject infected with HCV to a treatment with peginterferon alfa-2a, ribavirin and a direct acting antiviral agent comprising, providing a sample from the human subject and identifying the nucleotide present at single nucleotide polymorphism rs12148487, wherein the presence of at least one A allele at rs12148487 in the subject indicates a higher likelihood of RVR2 achieved by the subject to the treatment relative to a subject that has two G alleles present at rs12148487.
Figure 1. Study design of the NV21075 clinical trial. PEG-IFN = peginterferon alfa-2a; RBV = ribavirin; Q8h = tid or three times daily; Q 12h = bid or two times daily.
Figure 2. Percentage of patients with virological response up to Week 12.
Figure 3. Mean HCV RNA (log10 IU/ml) change from baseline to Week 12.
Figure 4. Percentage of CC patients and non-CC patients in the rs12979860 SNP with virological response up to Week 12.
Figure 5. Genome-wide association results for (A) RVR2 and (B) RVR2 after adjustment for rs12979860 by chromosome in the NV21075 study population.
Figure 6. Bar-plot showing the association between RVR2 and rs12148487genotype amongst the 4 treatment groups in the NV21075 clinical trial.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The term "response" to treatment with interferon is a desirable response to the administration of an agent. Virological endpoints include the following: "rapid virological response-2 weeks" (RVR2), defined as undetectable HCV RNA in serum (by Cobas® AmpliPrep/Cobas® Taqman® HCV Test v1.0, limit of detection 15 IU/mL) after 2 weeks of treatment; "rapid virological response-4 weeks" (RVR4), defined as undetectable HCV RNA in serum (by Cobas® AmpliPrep/Cobas® Taqman® HCV Test v1.0, limit of detection 15IU/mL) after 4 weeks of treatment; "early virological response" (EVR), defined as ?2-log drop in serum HCV RNA from baseline to week 12 (by Cobas® AmpliPrep/Cobas® Taqman® HCV Test, v1.0, limit of quantitation 43 IU/mL), complete EVR (cEVR) defined as undetectable HCV RNA in serum (by Cobas® AmpliPrep/Cobas® Taqman® HCV Test v1.0, limit of detection 15 IU/mL) after 12 weeks of treatment; and "sustained virological response" (SVR), defined as undetectable HCV RNA in serum (<15 IU/mL) at the end of a 12-week untreated follow-up period (SVR-12) or a 24-week untreated follow-up period (SVR-24). The term "extended rapid virological response" (eRVR) refers to undetectable HCV RNA (<15 IU/mL) during the period of between 4 weeks and 20 weeks of treatment.

The terms "sample" or "biological sample" refers to a sample of tissue or fluid isolated from an individual, including, but not limited to, for example, tissue biopsy, plasma, serum, whole blood, spinal fluid, lymph fluid, the external sections of the skin, respiratory, intestinal and genitourinary tracts, tears, saliva, milk, blood cells, tumors, organs. Also included are samples of in vitro cell culture constituents (including, but not limited to, conditioned medium resulting from the growth of cells in culture medium, putatively virally infected cells, recombinant cells, and cell components).

The terms "interferon" and "interferon-alpha" are used herein interchangeably and refer to the family of highly homologous species-specific proteins that inhibit viral replication and cellular proliferation and modulate immune response. Typical suitable interferons include, but are not limited to, recombinant interferon alpha-2b such as Intron® A interferon available from Schering Corporation, Kenilworth, N.J., recombinant interferon alpha-2a such as Roferon®-A interferon available from Hoffmann-La Roche, Nutley, N.J., recombinant interferon alpha-2C such as Berofor® alpha 2 interferon available from Boehringer Ingelheim Pharmaceutical, Inc., Ridgefield, Conn., interferon alpha-n1, a purified blend of natural alpha interferons such as Sumiferon® available from Sumitomo, Japan or as Wellferon® interferon alpha-n1 (INS) available from the Glaxo-Wellcome Ltd., London, Great Britain, or a consensus alpha interferon such as those described in U.S. Pat. Nos. 4,897,471 and 4,695,623 (especially Examples 7, 8 or 9 thereof) and the specific product available from Amgen, Inc., Newbury Park, Calif., or interferon alpha-n3 a mixture of natural alpha interferons made by Interferon Sciences and available from the Purdue Frederick Co., Norwalk, Conn., under the Alferon Tradename. The use of interferon alpha-2a or alpha-2b is preferred. Interferons can include pegylated interferons as defined below.

The terms "pegylated interferon", "pegylated interferon alpha" and "peginterferon" are used herein interchangeably and means polyethylene glycol modified conjugates of interferon alpha, preferably interferon alfa-2a and alfa-2b. Typical suitable pegylated interferon alpha include, but are not limited to, Pegasys® and Peg-Intron®.

The term "ribavirin" refers to the compound, 1-((2R,3R,4S,5R)-3,4-Dihydroxy-5-hydroxymethyl-tetrahydro-furan-2-yl)-1H-[1,2,4]triazole-3-carboxylic acid amide which is a synthetic, non-interferon-inducing, broad spectrum antiviral nucleoside analog and available under the names, Virazole® and Copegus®.

The current recommended first line treatment for patients with chronic hepatitis C is pegylated interferon alpha in combination with ribavirin for 48 weeks in patients carrying genotype 1 or 4 virus and for 24 weeks in patients carrying genotype 2 or 3 virus. Combined treatment with ribavirin was found to be more effective than interferon alpha monotherapy in patients who relapsed after one or more courses of interferon alpha therapy, as well as in previously untreated patients. However, ribavirin exhibits significant side effects including teratogenicity and carcinogenicity. Furthermore, ribavirin causes hemolytic anemia requiring dose reduction or discontinuation of ribavirin therapy in approximately 10 to 20% of patients, which may be related to the accumulation of ribavirin triphosphate in erythrocytes. Therefore, to reduce treatment cost and the incidence of adverse events, it is desirable to tailor the treatment to a shorter duration while not compromising efficacy. A shortened "duration of treatment" for genotype 1 patients with pegylated interferon alpha with ribavirin would be, for example, 24 weeks. A shortened duration of treatment for genotype 1 patients with pegylated interferon alpha with ribavirin in combination with a direct acting antiviral agent could be as short as 8 weeks, 12 weeks, or 16 weeks.

The terms "danoprevir", "RG7227", "RO5190591" and "ITMN-191" are used interchangeably and refer to the macrocyclic peptidomimetic inhibitor of the HCV NS3/4A protease, 4-Fluoro-1,3-dihydro-isoindole-2-carboxylic acid (Z)-(1S,4R,6S,14S,18R)-14-tert-butoxycarbonylamino-4-cyclopropanesulfonylaminocarbonyl-2,15-dioxo-3,16-diazatricyclo[14.3.0.0*4,6*]nonadec-7-en-18-yl ester.

Other HCV NS3 and NS3/4A protease inhibitors include, "boceprevir" or "SCH-503034": (1R,5S)-N-[3-amino-1(cyclobutylmethyl)-2,3-dioxopropyl]-3-[2(S)-[[[(1,1-dimethylethyl)amino]carbonyl]amino]-3,3-dimethyl-1-oxobutyl]-6,6-dimethyl-3-azabicyclo[3.1.0]hexan-(S)-carboxamide; and "telaprevir" or "VX-950" : (1S,3aR,6aS)-2-[(2S)-2-[[(2S)-cyclohexyl[(pyrazinylcarbonyl)amino]acetyl]amino]-3,3-dimethylbutanoyl]-N-[(1S)-1-[(cyclopropylamino)oxoacetyl]butyl]octahydrocyclopenta[c]pyrrole-1-carboxamide; "vaniprevir" or " MK-7009": (1R,21S,24S)-21-tert-Butyl-N-((1R,2R)-1-{[(cyclopropylsulfonyl)amino]carbonyl}-2-ethylcyclopropyl)-16,16-dimethyl-3,19,22-trioxo-2,18-dioxa-4,20,23-triazatetracyclo[21.2.1.1.0]heptacosa-6,8,10-triene-24-carboxamide; "MK-5172: (1aR,5S,8S,10R,22aR)-5-(1,1-dimethylethyl)-1,1a,3,4,5,6,9,10,18,19,20,21,22a-tetradecahydrol-14-methoxy-3,6-dioxo-8H-7,10-methanocyclopropa[18,19][1,10,3,6]dioxadiazacyclononadencino[11,12-b]quinoxaline-8-carboxylic acid; "TMC-435": (2R,13aR,14aR,16aS,Z)-N-(cyclopropylsulfonyl)-2-(2-(4-isopropylthiazol-2-yl)-7-methoxy-8-methylquinolin-4-yloxy)-6-methyl-5,16-dioxo-1,2,3,5,6,7,8,9,10,11,13a,14,14a,15,16,16a-hexadecahydrocyclopropa[g]pyrrolo[1,2-c][1,3,6]triazacyclopentadecine-14a-carboxamide; and "narlaprevir" or "SCH-900518" : (1R,2S,5S)-3-((S)-2-(3-(1-(tert-butylsulfonylmethyl)cyclohexyl)ureido)-3,3-dimethylbutanoyl)-N-((S)-1-(cyclopropylamino)-1,2-dioxoheptan-3-yl)-6,6-dimethyl-3-azabicyclo[3.1.0]hexane-2-carboxamide.

The term "triple therapy treatment" refers to a treatment regimen for HCV infected patients that comprises peginterferon, ribavirin and one or more direct acting antiviral agent. "Direct acting antiviral agents" exert specific antiviral effects independent of immune function. Examples of direct acting antiviral agents for HCV include but are not limited to NS3/4A protease inhibitors, NS5B polymerase inhibitors, NS5A inhibitors, IRES inhibitors and helicase inhibitors. One example of a triple therapy treatment comprises peginterferon, ribavirin, and a HCV NS5B polymerase inhibitor. Another example of a triple therapy treatment comprises peginterferon, ribavirin, and a HCV NS3/4A protease inhibitor. A further example of a triple therapy treatment comprises peginterferon, ribavirin, a HCV NS5B polymerase inhibitor, and a HCV NS3/4A protease inhibitor (which may also be referred to as "quadruple therapy treatment".

For patients with chronic hepatitis C (CHC) the current recommended first line treatment, referred as "standard of care" or "SOC", is pegylated interferon alpha in combination with ribavirin for a "duration of time" of 48 weeks in patients carrying genotype 1 or 4 virus and for 24 weeks in patients carrying genotype 2 or 3 virus. Recently in certain countries (e.g. the United States and member nations in the European Union), SOC for treatment-naïve patients with chronic hepatitis C genotype 1 virus infection has been recommended as combination therapy with an HCV protease inhibitor (either boceprevir or telaprevir) and pegylated interferon alpha (PEG-IFN) plus ribavirin (RBV) (Ghany MG et al, "An Update on Treatment of Genotype 1 Chronic Hepatitis C Virus Infection: 2011 Practice Guideline by the American Association for the Study of Liver Diseases." Hepatology 2011; 54: 1433-1444). However, the term "SOC" as used in this document refers to treatment with PEG-IFN plus RBV only.

Combined treatment with ribavirin was found to be more effective than interferon alpha monotherapy in patients who relapsed after one or more courses of interferon alpha therapy, as well as in previously untreated patients. However, ribavirin exhibits significant side effects including teratogenicity and carcinogenicity. Furthermore, ribavirin causes hemolytic anemia requiring dose reduction or discontinuation of ribavirin therapy in approximately 10 to 20% of patients, which may be related to the accumulation of ribavirin triphosphate in erythrocytes. Therefore, to reduce treatment cost and the incidence of adverse events, it is desirable to tailor the treatment to a shorter duration while not compromising efficacy.

As used herein, the terms "allele" and "allelic variant" refer to alternative forms of a gene including introns, exons, intron/exon junctions and 3? and/or 5? untranslated regions that are associated with a gene or portions thereof. Generally, alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides.

As used herein, the term "polymorphism" refers to the coexistence of more than one form of a nucleic acid (e.g., allelic variant) thereof. A portion of a gene of which there are at least two different forms, i.e., two different nucleotide sequences, is referred to as a polymorphic region of a gene. A polymorphic region can be a single nucleotide, i.e. "single nucleotide polymorphism" or "SNP", the identity of which differs in different alleles. A polymorphic region can also be several nucleotides long.

Numerous methods for the detection of polymorphisms are known and may be used in conjunction with the present invention. Generally, these include the identification of one or more mutations in the underlying nucleic acid sequence either directly (e.g. allele-specific PCR) or indirectly (identifying changes to a secondary molecule, e.g., protein sequence).

One well-known method for genotyping single nucleotide polymorphisms is using the Illumina SNP beadchips where fragmented and denatured DNA samples are hybridized to the corresponding 50-mer oligos attached to the beads on the chip. After hybridization the chips are processed for enzymatic single base extension followed by fluorescent staining and imaging. Finally, fluorescence intensities are detected and used for SNP genotype calling.

The single nucleotide polymorphisms, "rs12979860" "rs12980275" and "rs8099917" refer to SNPs identified by their accession numbers in the database of SNPs (dbSNP, www.ncbi.nlm.nih.gov/SNP/) and are located on human chromosome 19 in various regions around the IL28b gene.

The single nucleotide polymorphism "rs12148487" refers to the SNP identified by its accession number in dbSNP and is located on human chromosome 15 in the intronic region of the SLCO3A1 gene which is a member of the solute carrier organic anion transporter family. Terms that are synonyms for SLCO3A1 include OATP-D, OATP3A1. OATPD, and SLC21A11.

### EXAMPLES

### Introduction

The HCV protease, encoded by the nonstructural gene 3/4A (NS3/4A), represents a clinically validated anti-HCV target and is essential for HCV polypeptide post-translational modification and viral replication. Danoprevir, also known as RO5190591, RG7227 or ITMN-191, is a macrocyclic peptidomimetic compound that competitively inhibits the HCV NS3/4A protease. Danoprevir was selected for development as an oral agent for the treatment of patients with chronic HCV (CHC) because of its high antiviral potency, specificity, and safety profile.

### Study design

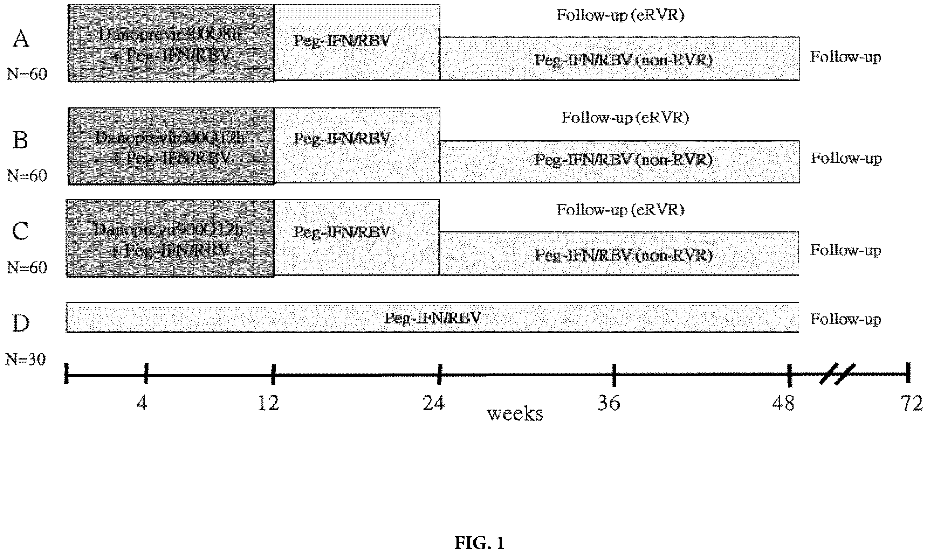

Study NV21075 was a randomized double-blinded multicenter Phase 2 clinical study to evaluate the efficacy and safety of danoprevir in combination with peginterferon alfa 2a and ribavirin (triple therapy combination) when dosed for twelve weeks in treatment-naïve patients with chronic Hepatitis C genotype 1 infection as compared to the currently approved combination of peginterferon alfa 2a and ribavirin (Standard of Care or SOC). Patients enrolled in Study NV21075 were randomized into one of four treatment groups (Figure 1) (approximately 60 patients for Groups A-C, approximately 30 patients for Group D). In the response-guided treatment groups (as described below), patients with an extended RVR (eRVR, defined as undetectable HCV RNA from week 4-20) stopped all therapy at Week 24.

Treatment Groups A, B, C: Patients received double blinded experimental treatment with danoprevir at 300 mg TID (Group A), 600 mg BID (Group B), or 900 mg BID (Group C) and standard of care treatment (SOC, Pegasys 180 g sc qw + Copegus 1000 mg (<75 kg) or 1200 mg (?75 kg) po qd) for 12 weeks, followed by SOC for 12 weeks. Patients who achieve an RVR, defined as undetectable HCV RNA at week 4, and remain undetectable through week 22 (i.e. achieving eRVR) stopped all treatement at week 24. Patients that did not achieve eRVR received SOC for an additional 24 weeks for a total treatment duration of 48 weeks. Treatment Group D: Patients received double blinded placebo treatment for 12 weeks, followed by SOC for 36 weeks for a total duration of 48 weeks.

### RCR patient population with IL28B genotyping and genome wide association study (GWAS)

The Roche Clinical Repository (RCR) is an optional program that requires separate consent; data are anonymized to assure patient privacy. The RCR DNA samples were used to determine IL28B status using TaqMan® Real Time PCR for rs12979860. Efficacy endpoints were summarized for the two subgroups, CC vs. non-CC (CT and TT). For the NV21075 study, genotyping data was available from 171 patient samples. In addition, these RCR DNA samples were used for GWAS and were genotyped by the Illumina Human OmniExpress Beadchip with a total number of 733,202 single nucleotide polymorphisms (SNPs). 725,947 SNPs out of 733,202 passed quality control (QC). Genome wide association analysis was performed in 166 samples with complete clinical dataset.

### Results

For the interim analysis of NV21075, efficacy during the first 12 weeks of treatment was measured by the percentage of patients with virological response (undetectable HCV RNA as measured by the Roche Cobas® AmpliPrep/Cobas® Taqman® HCV Test v1.0 [limit of detection 15 IU/mL]). Patients were considered non-responders at a scheduled visit if they had missing data, which may have been due to an assessment that was missed or fell outside of the allowable window for that visit, or an early withdrawal from treatment with a missing follow-up assessment.

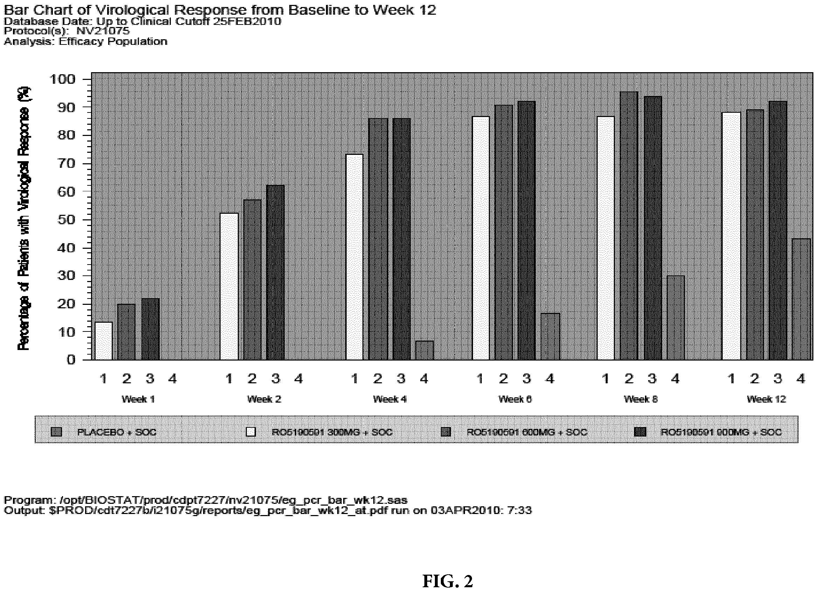

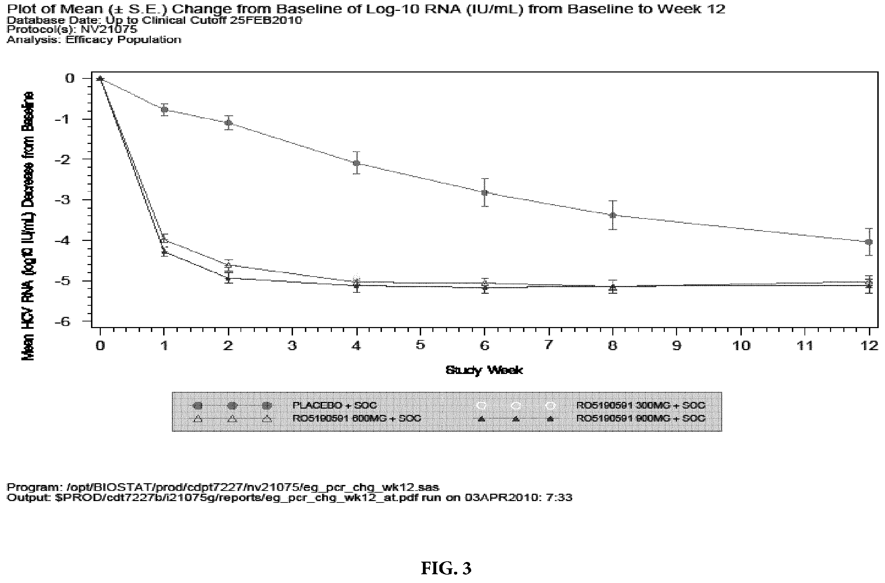

Preliminary results indicate that 88.1%, 89.2%, and 92.0% of patients in Treatment Groups A, B, and C, respectively, had undetectable HCV RNA levels at the end of 12 weeks of treatment compared with 43.3% of patients who received SOC (Figure 2). Danoprevir-treated patients experienced a precipitous decline in mean HCV RNA levels within the first 2 to 4 weeks of treatment and remained consistently lower compared with patients who received SOC up to Week 12 (Figure 3).

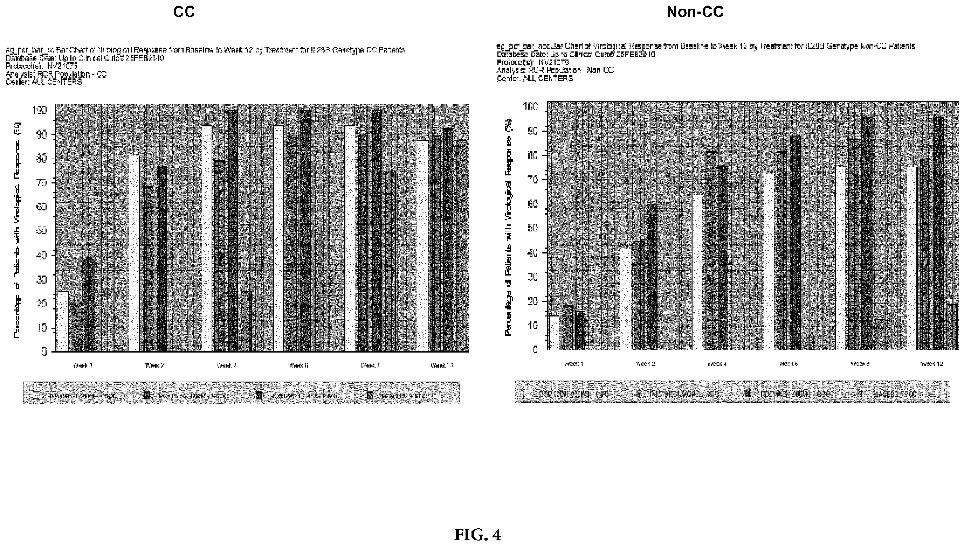

For the 171 patient samples in which genotyping data was available for the rs12979860 SNP in the IL28B gene, 56 patients possessed the CC (treatment favorable) genotype while 115 patients carried the non-CC (treatment unfavorable) genotype. Regardless of which treatment group they were in, CC patients showed better virologic response rates than non-CC patients, particularly within the first two weeks of treatment (Figure 4).

In the subset of patients from Treatment Groups A and B for which HCV RNA levels at 12 weeks of post-treatment follow-up (i.e. 12 weeks after cessation of treatment) were available, the correlations between those who achieved the early measures of response, RVR2, RVR4, eRVR, and those who achieved SVR-12 were determined. Among 68 patients in Treatment Groups A and B who achieved RVR2, 64 patients achieved SVR-12, which yielded a positive predictive value (PPV) of RVR2 for SVR-12 of 94%. In comparison, out of the 97 patients who achieved RVR4, 87 patients achieved SVR-12 yielding a PPV of RVR4 for SVR-12 of 90%. The PPV of eVR for SVR-12 was also 90% as 84 patients among the 93 patients who achieved eRVR also achieved SVR-12. These results showed that the early measures of response, especially RVR2, are highly correlated with SVR-12.

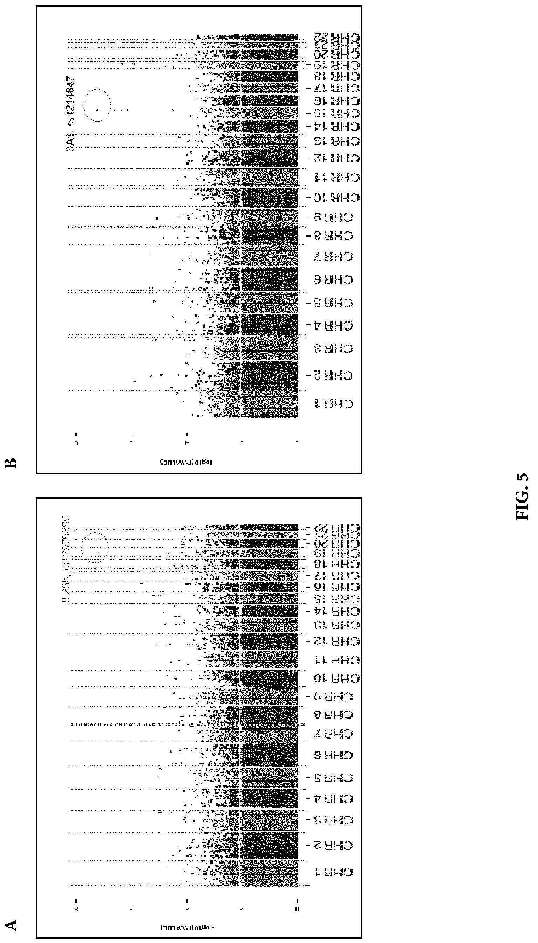

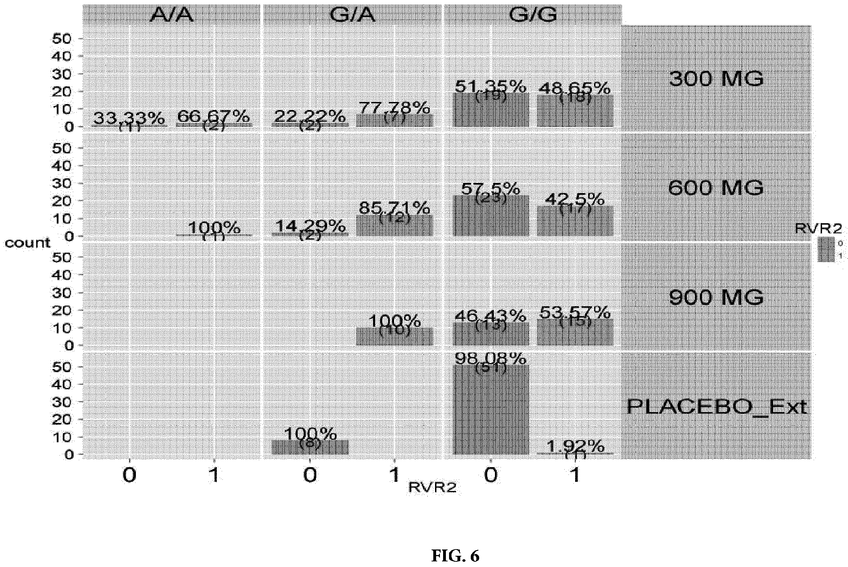

In order to identify additional single nucleotide polymorphisms that may be associated with clinical efficacy endpoints in HCV treatment in response to danoprevir plus SOC triple therapy or with SOC therapy alone, patient samples were genotyped on the Illumina OmniExpress chipset. After quality control, 725,947 SNPs with genotype calls were generated. A logistic regression model was used to test for associations between individual SNPs and Rapid Virological Response-2 Weeks (RVR2) after adjustment for viral load at baseline and sample ethnicity. Not surprisingly, the rs12979860 SNP showed marked association with RVR2 with p value of 6 x 10-8 (Figure 5A). After adjustment for rs12979860, additional SNPs on chromosome 15 with p<10-5 were observed (Figure 5B) with the most prominent SNP being rs12148487 which is in the intron of the gene coding for the solute carrier organic anion transporter, SLCO3A1. In the NV21075 population, the association between RVR2 and the rs12148487 minor allele, A, is seen with an Odds Ratio (OR) of over 21(OR = 21.87, 95% CI [6.30; 96.88], p=5.6 x 10-8). These results were obtained using a dominant model in the logistic regression for rs12148487, with AA and GA genotypes coded '1' and GG genotype coded '0'. Figure 6 shows the bar-plot of the association between RVR2 and rs12148487.

All of the compositions and/or methods disclosed and claimed herein can be made and executed without undue experimentation in light of the present disclosure.

### REFERENCES

1. Lavanchy, D. Global Surveillance and Control of Hepatitis C: Report of a WHO Consultation. J Viral Hepatitis. 1999;6:35-47.
2. Consensus Statement: EASL/International Consensus Conference on Hepatitis C. J Hepatol. 1999;31(Suppl 1):3 8.
3. Recommendations from the National Institutes of Health Consensus Development Conference Statement: Management of Hepatitis C: 2002. Hepatology Nov 2002; Vol 36 No 5:1039.
4. Fried MW, Shiffman ML, Reddy KR, Smith C, Marinos G, Goncales FL Jr, et al. 2002. Peginterferon alfa-2a plus rivavirin for chronic hepatitis C virus. NEJM September 26 2002; Vol 347 No 13:975-982
5. Hadziyannis SJ, Sette Jr. H, Morgan TR et al. Peginterferon alfa-2a and ribavirin combination therapy in chronic hepatitis C. Ann Int Med 2004 Vol 140:346-355.
6. Manns MP, McHutchison JG, Gordon SC, Rustgi VK, Shiffman M, Reindollar R, Goodman ZD, Koury K, Ling M, Albrecht JK. Peginterferon alfa-2b plus ribavirin compared with interferon alfa-2b plus ribavirin for initial treatment of chronic hepatitis C: a randomised trial. Lancet 2001;358:958-965.
7. Report No. 1022744; 2006: Inhibition of RNA synthesis by by ?-D-2 -deoxy-2 -fluoro-2 -C-methylcytidine (RO4995855) in the HCV Subgenomic Replicon System. Roche Palo Alto LCC.
8. RO5024048 Investigator s Brochure, November 2008.
9. PEG-IFN alfa-2a Investigator s Brochure, July, 2006.
10. PEGASYS Core Data Sheet Version 1.4, January 19, 2005.
11. Sidwell RW, Robins RK, and Hillyard IW. Ribavirin: an antiviral agent. Pharmacol Ther 1979; 6:123-146.
12. Gilbert BE and Knight V. Minireview: Biochemistry and clinical applications for ribavirin. Antimicrob Agents Chemother. 1986; 30:201-205.
13. Dusheiko G, Main J, Thomas H et al. Ribavirin treatment for patients with chronic hepatitis C: Results of a placebo-controlled trial. J Hepatol 1996; 25:591-598.
14. Di Bisceglie AM, Conjeevaram HS, Fried MW et al. Ribavirin as therapy for chronic hepatitis C. A randomized, double-blind, placebo-controlled trial. Ann Int Med 1995; 123:897-903.
15. Conjeevaram HS, Fried MW, Jeffers LJ, Terrault NA, Wiley-Lucas TE, Afdhal N, Brown RS, Belle SH, Hoofnagle JH, Kleiner DE, Howell CD. Peginterferon and ribavirin treatment in African American and Caucasian American patients with hepatitis C genotype 1. Gastroenterology 2006;131:470-477.
16. Dienstag JL, McHutchison JG. American Gastroenterological Association technical review on the management of hepatitis C. Gastroenterology 2006;130:231-264.
17. Missiha S, Heathcote J, Arenovich T, Khan K. Impact of asian race on response to combination therapy with peginterferon alfa-2a and ribavirin in chronic hepatitis C. Am J Gastroenterol 2007;102:2181-2188.
18. Reddy KR, Messinger D, Popescu M, Hadziyannis SJ. Peginterferon alpha-2a (40 kDa) and ribavirin: comparable rates of sustained virological response in sub-sets of older and younger HCV genotype 1 patients. J Viral Hepat 2009;16:724-731.
19. Ferenci P, Fried MW, Shiffman ML, Smith CI, Marinos G, Goncales FL, Jr., Haussinger D, Diago M, Carosi G, Dhumeaux D, Craxi A, Chaneac M, Reddy KR. Predicting sustained virological responses in chronic hepatitis C patients treated with peginterferon alfa-2a (40 KD)/ribavirin. J Hepatol 2005;43:425-433.
20. Martinot-Peignoux M, Maylin S, Moucari R, Ripault MP, Boyer N, Cardoso AC, Giuily N, Castelnau C, Pouteau M, Stern C, Auperin A, Bedossa P, Asselah T, Marcellin P. Virological response at 4 weeks to predict outcome of hepatitis C treatment with pegylated interferon and ribavirin. Antivir Ther 2009;14:501-511.
21. Asselah T, Bieche I, Sabbagh A, Bedossa P, Moreau R, Valla D, Vidaud M, Marcellin P. Gene expression and hepatitis C virus infection. Gut 2009;58:846-858.
22. Ge D, Fellay J, Thompson AJ, Simon JS, Shianna KV, Urban TJ, Heinzen EL, Qiu P, Bertelsen AH, Muir AJ, Sulkowski M, McHutchison JG, Goldstein DB. Genetic variation in IL28B predicts hepatitis C treatment-induced viral clearance. Nature 2009;461:399-401.
23. Suppiah V, Moldovan M, Ahlenstiel G, Berg T, Weltman M, Abate ML, Bassendine M, Spengler U, Dore GJ, Powell E, Riordan S, Sheridan D, Smedile A, Fragomeli V, Muller T, Bahlo M, Stewart GJ, Booth DR, George J. IL28B is associated with response to chronic hepatitis C interferon-alpha and ribavirin therapy. Nat Genet 2009;41:1100-1104.
24. Tanaka Y, Nishida N, Sugiyama M, Kurosaki M, Matsuura K, Sakamoto N, Nakagawa M, Korenaga M, Hino K, Hige S, Ito Y, Mita E, Tanaka E, Mochida S, Murawaki Y, Honda M, Sakai A, Hiasa Y, Nishiguchi S, Koike A, Sakaida I, Imamura M, Ito K, Yano K, Masaki N, Sugauchi F, Izumi N, Tokunaga K, Mizokami M. Genome-wide association of IL28B with response to pegylated interferon-alpha and ribavirin therapy for chronic hepatitis C. Nat Genet 2009;41:1105-1109.

## Claims

1. A method for predicting response of a human subject infected with HCV to a treatment with polyethylene glycol modified conjugates of interferon alfa-2a, ribavirin and a direct acting antiviral agent comprising:
identifying the nucleotide present at single nucleotide polymorphism rs12148487 in a sample from said human subject, wherein the presence of at least one A allele at rs 12148487 in said subject indicates a higher likelihood of rapid virological response in two weeks (RVR2) achieved by said subject to said treatment relative to a subject that has two G alleles present at rs 12148487.

2. The method of claim 1 wherein said subject is infected with HCV Genotype-1 or HCV Genotype-4.

3. The method of claim 2 wherein said direct acting antiviral agent is a HCV protease inhibitor.

4. The method of claim 3 wherein said HCV protease inhibitor is selected from a group consisting of danoprevir, boceprevir, telaprevir, vaniprevir, MK-5172, TMC-435, and narlaprevir.

5. The method of claim 4 wherein said HCV protease inhibitor is danoprevir.

## Patentansprüche

1. Verfahren zum Prognostizieren des Ansprechens eines humanen Individuums, das mit HCV infiziert ist, auf eine Behandlung mit Polyethylenglykol-modifizierten Konjugaten von Interferon alfa-2a, Ribavirin und einem direkt wirkenden antiviralen Agens, umfassend:
Identifizieren des Nucleotids, das am Einzelnucleotid-Polymorphismus (single nucleotide polymorphism) rs12148487 in einer Probe des humanen Individuums anwesend ist, wobei die Anwesenheit von mindestens einem A-Allel an rs12148487 in dem Individuum auf eine höhere Wahrscheinlichkeit einer raschen virologischen Antwort innerhalb von zwei Wochen (rapid virological response; RVR2) hinweist, die von dem Individuum auf die Behandlung erreicht wurde, verglichen mit einem Individuum, das zwei G-Allele an rs12148487 hat.

2. Verfahren nach Anspruch 1, wobei das Individuum mit HCV vom Genotyp 1 oder HCV vom Genotyp 4 infiziert ist.

3. Verfahren nach Anspruch 2, wobei das direkt wirkende antivirale Agens ein HCV-Protease-Inhibitor ist.

4. Verfahren nach Anspruch 3, wobei der HCV-Protease-Inhibitor ausgewählt ist aus einer Gruppe bestehend aus Danoprevir, Boceprevir, Telaprevir, Vaniprevir, MK-5172, TMC-435 und Narlaprevir.

5. Verfahren nach Anspruch 4, wobei der HCV-Protease-Inhibitor Danoprevir ist.

## Revendications

1. Méthode de prédiction de la réponse d'un sujet humain infecté par le VHC à un traitement avec des conjugués modifiés avec du polyéthylène glycol composés de l'interféron alfa-2a, de la ribavirine et d'un agent antiviral à action directe, comprenant :
l'identification du nucléotide présent au niveau du polymorphisme mononucléotidique rs 12148487 dans un échantillon provenant dudit sujet humain, dans laquelle la présence d'au moins un allèle A au niveau de rs12148487 chez ledit sujet indique une probabilité plus élevée de réponse virologique rapide en deux semaines (RVR2) obtenue par ledit sujet audit traitement par rapport à un sujet qui a deux allèles G présents au niveau de rs 12148487.

2. Méthode selon la revendication 1, dans laquelle ledit sujet est infecté par le VHC de génotype 1 ou le VHC de génotype 4.

3. Méthode selon la revendication 2, dans laquelle ledit agent antiviral à action directe est un inhibiteur de protéase de VHC.

4. Méthode selon la revendication 3, dans laquelle ledit inhibiteur de protéase de VHC est choisi dans le groupe constitué du danoprévir, du bocéprévir, du télaprévir, du vaniprévir, du MK-5172, du TMC-435 et du narlaprévir.

5. Méthode selon la revendication 4, dans laquelle ledit inhibiteur de protéase de VHC est le danoprévir.
